Europäisches Patentamt

European Patent Office

Office européen des brevets

(11) Numéro de publication : **0 493 249 A1**

(12) # DEMANDE DE BREVET EUROPEEN

(21) Numéro de dépôt : **91403530.8**

(22) Date de dépôt : **24.12.91**

(51) Int. Cl.⁵ : **G03B 42/02, H01J 35/06, G21K 1/02**

(30) Priorité : **28.12.90 FR 9016464**

(43) Date de publication de la demande :
**01.07.92 Bulletin 92/27**

(84) Etats contractants désignés :
**DE ES GB IT NL**

(71) Demandeur : **GENERAL ELECTRIC CGR S.A.**
**100, rue Camille-Desmoulins**
**F-92130 Issy les Moulineaux (FR)**

(72) Inventeur : **Evain, Bernard**
**Cabinet Ballot-Schmit, 7 rue Le Sueur**
**F-75116 Paris (FR)**
Inventeur : **Peyret, Olivier**
**Cabinet Ballot-Schmit, 7 rue Le Sueur**
**F-75116 Paris (FR)**
Inventeur : **Dumitrescu, Horia**
**Cabinet Ballot-Schmit, 7 rue Le Sueur**
**F-75116 Paris (FR)**

(74) Mandataire : **Ballot, Paul Denis Jacques et al**
**Cabinet Ballot-Schmit, 7, rue le Sueur**
**F-75116 Paris (FR)**

(54) **Procédé, tube et système pour éliminer une grille anti-diffusante fixe dans une image radiologique.**

(57) L'invention concerne les appareils radiologiques utilisant une grille anti-diffusante.

L'invention réside dans le fait que la grille anti-diffusante est maintenue fixe au lieu d'être mobile et que son image est éliminée en l'irradiant ainsi que l'objet à examiner par deux sources de rayons X qui doivent être séparées par une distance b déterminée par le premier zéro de la fonction de transfert de modulation (33) de ce système à deux sources. Pour tenir compte du grandissement G, la distance b est réglable grâce à une cathode émettant deux faisceaux d'électrons à déviation réglable.

FIG. 6

EP 0 493 249 A1

L'invention concerne, d'une manière générale, les appareils radiologiques qui réalisent l'image d'un objet par l'utilisation d'un rayonnement X qui irradie ledit objet; elle concerne, de manière plus particulière, un procédé, un tube à rayons X et un système pour éliminer, dans l'image dudit objet, celle correspondant à une grille anti-diffusante fixe.

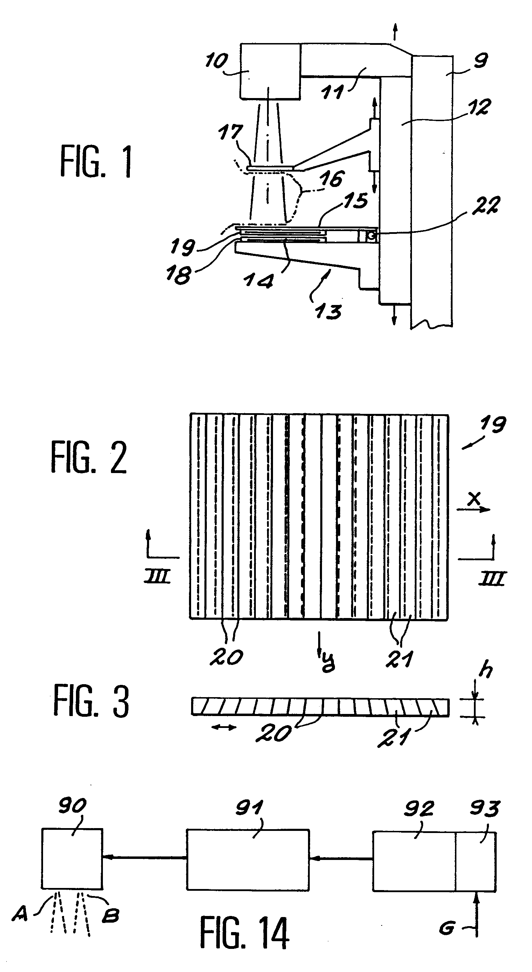

L'invention sera décrite dans son application à un appareil de mammographie du type de celui schématisé sur la figure 1.

Il comprend une source 10 de rayonnement X portée par une potence 11 disposée au sommet d'une plaque verticale 12. Cette dernière comporte un ensemble 13 sur lequel repose un sein 16 à examiner par l'intermédiaire d'une tablette horizontale 15. Une pelote 17, transparente au rayonnement X et mobile verticalement sur la plaque 12, sert à comprimer le sein.

Pour s'adapter à la taille de la patiente, la plaque 12 est montée sur une colonne verticale 9 reposant sur le sol et se déplace verticalement sur ladite colonne grâce à un dispositif mécanique approprié.

L'ensemble 13 présente, sur sa partie supérieure et sous la tablette 15, un tunnel dans lequel vient se loger une cassette 18 constituée d'une boîte noire renfermant un film sensible 14 au rayonnement X direct ou à un rayonnement photonique émis par un écran (non représenté) recevant le rayonnement X. C'est sur ce film 14 que se forme l'image latente du sein après une durée de pose appropriée; le développement du film donne un cliché radiographique.

Dans un mammographe, le sein 16 en cours d'examen est proche du film radiologique 14 et, par suite de la diffusion réalisée par les différents points du sein, le contraste et la résolution de l'image sont diminués.

Pour éviter ce phénomène, on interpose entre le sein et le film radiologique une grille 19, dite anti-diffusante, qui élimine tous les rayons diffusés ayant un angle d'incidence sur le film 14 supérieur à une certaine valeur.

Une telle grille comporte (figures 2 et 3) une succession de lames de plomb 20, matériau absorbant pour les rayons X, séparées par des intercalaires 21 en matériau peu absorbant pour les rayons X, tel que du papier ou de l'aluminium. Ces lames sont orientées de manière convergente vers le foyer du tube à rayons X.

Typiquement, les lamelles ont une épaisseur d = 10 à 30 microns pour un pas de D = 100 à 300 microns et une hauteur h de quelques millimètres.

Comme une telle grille comporte des lames opaques au rayonnement X, ces lames donnent chacune une image sur le film radiologique. Aussi, pour éliminer cette image de la grille, il est connu d'animer cette dernière d'un mouvement alternatif de quelques hertz qui la rend invisible sur l'image radiologique. Ce mouvement alternatif est en général obtenu par un moteur 22, ce qui augmente l'encombrement de l'ensemble.

Un but de la présente invention est donc de mettre en oeuvre un procédé qui permet d'utiliser une grille fixe et d'éliminer l'image de la grille sur le film radiologique.

Ce procédé consiste à irradier l'objet par deux rayonnements X simultanés obtenus à l'aide d'un tube à rayons X à deux foyers identiques de largeur a espacés d'une distance b qui est choisie de manière à obtenir l'élimination de l'image de la grille.

La distance b est déterminée à partir du calcul de la fonction de transfert de modulation d'un tube à rayons X à deux foyers.

De manière plus précise, dans un appareil radiologique comportant au moins une source de rayons X irradiant un objet à examiner qui est situé à une distance c de ladite source et une grille anti-diffusante de fréquence spatiale $F_g$ disposée entre ledit objet et un récepteur situé à une distance d dudit objet, l'invention concerne un procédé permettant d'éliminer l'image de ladite grille anti-diffusante qui consiste :

– à maintenir fixe la grille anti-diffusante,

– à irradier simultanément ledit objet par une deuxième source de rayons X ayant une répartition énergétique spatiale sensiblement identique à celle de la première source, et

– à choisir la distance b entre les deux sources de rayons X pour que

$$b = \frac{G}{G-1} \cdot \frac{1}{2\,F_g} \quad (1)$$

avec le grandissement

$$G = \frac{c + d}{c} \quad (2)$$

Dans le cas où le grandissement G varie, il faut en outre modifier la distance b.

Les deux sources de rayonnement X sont réalisées à l'aide d'un tube à rayons X caractérisé en ce qu'il comprend une cathode comportant deux filaments, chaque filament étant associé à un dispositif de focalisation et de déviation dont les caractéristiques mécaniques et électriques ainsi que les tensions appliquées sont telles qu'il donne naissance, sur une anode disposée en face de ladite cathode, à deux sources de rayons X séparées par une distance b, les deux sources de rayons X ainsi obtenues ayant une répartition énergétique spatiale aussi identique que possible.

Les deux filaments de la cathode peuvent être identiques ou non mais, dans les deux cas, des étalonnages du tube à rayons X permettent de déterminer les tensions à appliquer aux dispositifs de focalisation et de déviation pour obtenir les deux foyers de même largeur a et d'espacement b en fonction du grandissement G.

D'autres buts, avantages et caractéristiques de la présente invention apparaîtront à la lecture de la description suivante d'un exemple particulier de réalisation, ladite description étant faite avec les dessins joints dans lesquels :

– la figure 1 est une vue en coupe schématique d'un mammographe auquel s'applique plus particulièrement l'invention,

– la figure 2 est une vue de dessus d'une grille anti-diffusante utilisée dans un mammographe,

– la figure 3 est une vue en coupe d'une grille anti-diffusante selon la ligne III-III de la figure 2,

– la figure 4 est un diagramme représentant les positions respectives du foyer, de l'objet, de la grille anti-diffusante et du film radiologique, dans un appareil radiologique,

– la figure 5 est un diagramme représentant la répartition énergétique théorique de deux foyers voisins et leur position respective,

– la figure 6 sont des diagrammes permettant de déterminer la courbe de fonction de transfert de modulation d'un émetteur de rayons X comportant deux foyers identiques,

– la figure 7 est un diagramme analogue à celui de la figure 6 mais avec un axe des abscisses gradué,

– la figure 8 est un diagramme donnant la courbe de la fonction de transfert de modulation d'un récepteur de rayons X tel qu'un film radiologique,

– la figure 9 est un diagramme donnant la courbe de la fonction de transfert de modulation d'une chaîne image comportant un émetteur à deux foyers et un récepteur,

– les figures 10, 11 et 12 sont des schémas de cathode d'un tube à rayons X qui permettent d'obtenir deux foyers identiques dont l'espacement peut être variable.

– les figures 13-a, 13-b et 13-c sont des diagrammes qui permettent de déterminer les tensions à appliquer aux électrodes de focalisation et de déviation pour obtenir des foyers ayant des caractéristiques souhaitées, et

– la figure 14 est un schéma fonctionnel simplifié d'un système selon l'invention.

Afin d'éliminer l'image d'une grille anti-diffusante de fréquence spatiale $F_g$ sur un film radiologique, l'invention propose d'utiliser un tube à rayons X qui comporte deux foyers qui ont une largeur a et un espacement b tels que la fonction de transfert de modulation présente une fréquence de coupure pour la fréquence spatiale Fg.

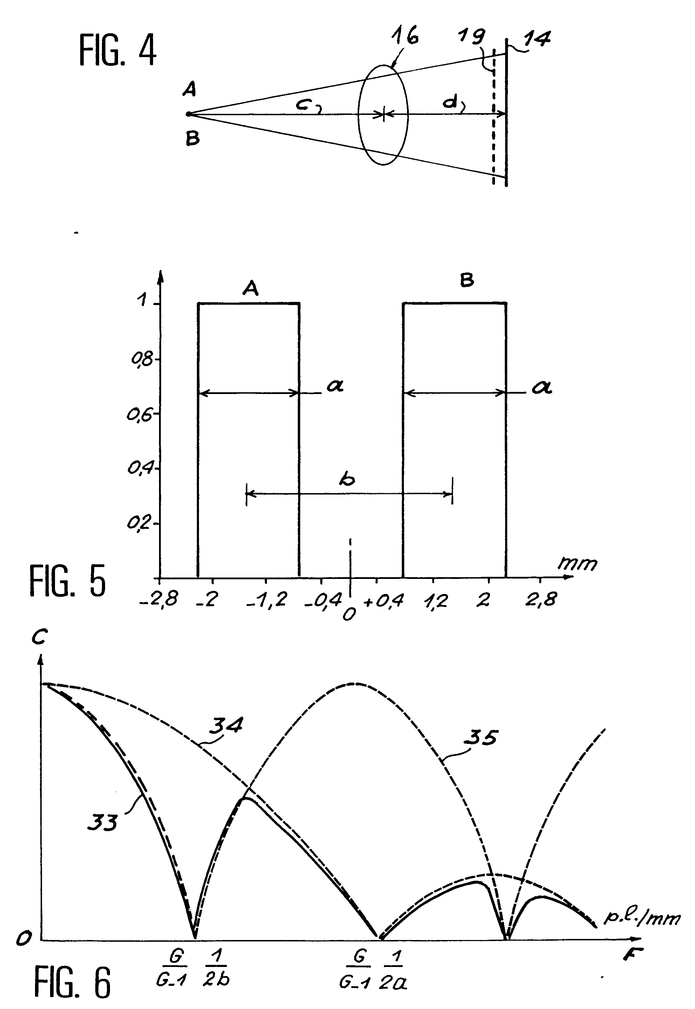

La manière dont une telle élimination est obtenue sera expliquée à l'aide des diagrammes des figures 4 à 9.

Le diagramme de la figure 4 indique les positions respectives des foyers A et B, d'un objet à examiner tel que le sein 16, de la grille anti-diffusante 19 et d'un récepteur tel que film radiologique 14. Si c est la distance entre les foyers et le sein 16 et d la distance entre le sein 16 et le film 14, on définit le grandissement G comme le rapport

$$G = \frac{c + d}{c}$$

La figure 5 donne la répartition énergétique théorique des foyers A et B en fonction de leur abscisse par rapport à un point milieu 0. Pour chaque foyer, cette répartition a une forme rectangulaire qui est supposée parfaite.

La fonction de transfert de modulation des deux foyers A et B présentant la répartition énergétique du diagramme de la figure 5, qui est donnée par le module de la fonction de transfert optique, est représentée par la courbe 33 en trait plein de la figure 6. Sur cette figure 6, l'axe des ordonnées est gradué en valeurs de contraste de 0 à 1 définies comme le rapport C tel que :

$$C = \frac{I_{max} - I_{min}}{I_{max} - I_{min}}$$

$I_{max}$ étant la valeur maximum et $I_{min}$ étant la valeur minimum de l'intensité lumineuse.

L'axe des abscisses en gradué en fréquence spectrale F et, plus précisément, en paires de lignes par millimètre, ce qui pour une grille anti-diffusante signifie le nombre de paires de lames opaques par millimètre (p.l./mm).

Cette courbe 33 est le produit des deux courbes 34 et 35 en tirets. La courbe 34 est du type module de :

$$\frac{\sin x}{x}$$

et ne dépend que de la largeur a des foyers A et B. La courbe 35 est du type module de (cos y) et ne dépend que l'espacement b des foyers A et B.

Eu égard au fait que b est en général supérieur à a, la première valeur de la fréquence spatiale pour laquelle le contraste s'annule est donné par :

$$\frac{G}{G-1} \cdot \frac{1}{2b}$$

c'est-à-dire par la courbe du type $|\cos y|$.

D'une manière générale, les fréquences spatiales pour lesquelles $|\cos y| = 0$ sont données par :

$$F_{oy} = (2k + 1) \cdot \frac{G}{G-1} \cdot \frac{1}{2b} \text{ avec } k \geqq 0 \quad (4)$$

La deuxième valeur de la fréquence spatiale pour laquelle le contraste s'annule est donnée par

$$\frac{G}{G-1} \cdot \frac{1}{a} \quad (5)$$

c'est-à-dire par la courbe du type

$$\left| \frac{\sin x}{x} \right|$$

dans le cas où b < 3a

D'une manière générale, les fréquences spatiales pour lesquelles

$$\left| \frac{\sin x}{x} \right| = 0$$

sont données par :

$$F_{ox} = 2k \cdot \frac{G}{G-1} \cdot \frac{1}{2a} \text{ avec } k \geqq \underline{1} \quad (6)$$

La courbe résultante 33, qui est le produit des courbes 34 et 35, montre que pour éliminer dans l'image un objet ayant une fréquence spatiale $F_g$, il faut choisir, pour un grandissement G donné, une valeur de l'espacement b tel que :

$$F_g = \frac{G}{G-1} \cdot \frac{1}{2b} \quad (7)$$

soit

$$b = \frac{G}{G-1} \cdot \frac{1}{2F_g} \quad (1)$$

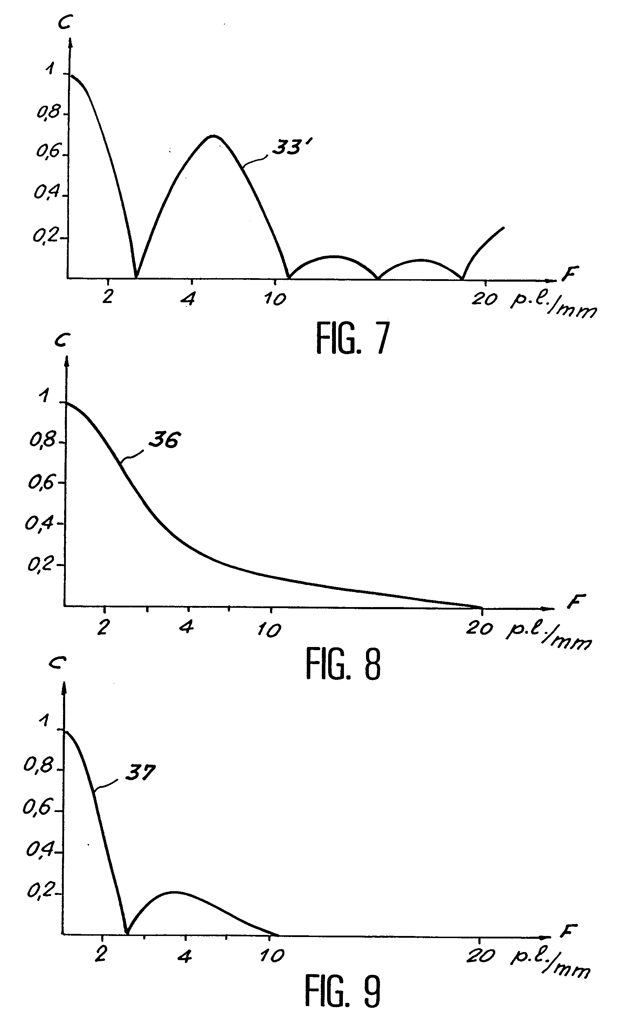

La fonction de transfert de modulation des foyers A et B telle que représentée par la courbe 33 de la figure 6 ou la courbe 33′ de la figure 7 ne tient pas compte de la fonction de transfert de modulation du récepteur représentée par la courbe 36 de la figure 8. S'il en est tenu compte, la fonction de transfert de modulation de la chaîne image est représentée par la courbe 37 de la figure 9 qui est le produit des courbes 33′ et 36.

La courbe 33′ de la figure 7 est similaire à la courbe 33 de la figure 6 mais elle a été tracée avec la même échelle des abscisses que celle des figures 8 et 9. Les tubes à rayons X qui comportent deux foyers sont connus mais les caractéristiques de chacun des foyers sont en général différentes car on souhaite obtenir des rayonnements X ayant des caractéristiques différentes.

Pour réaliser un tube à rayons X qui présente deux foyers simultanés, il faut utiliser deux sources d'émission d'électrons, c'est-à-dire deux filaments émissifs. Ceci peut être obtenu de différentes manières, par exemple par deux cathodes séparées comportant chacune un filament émissif.

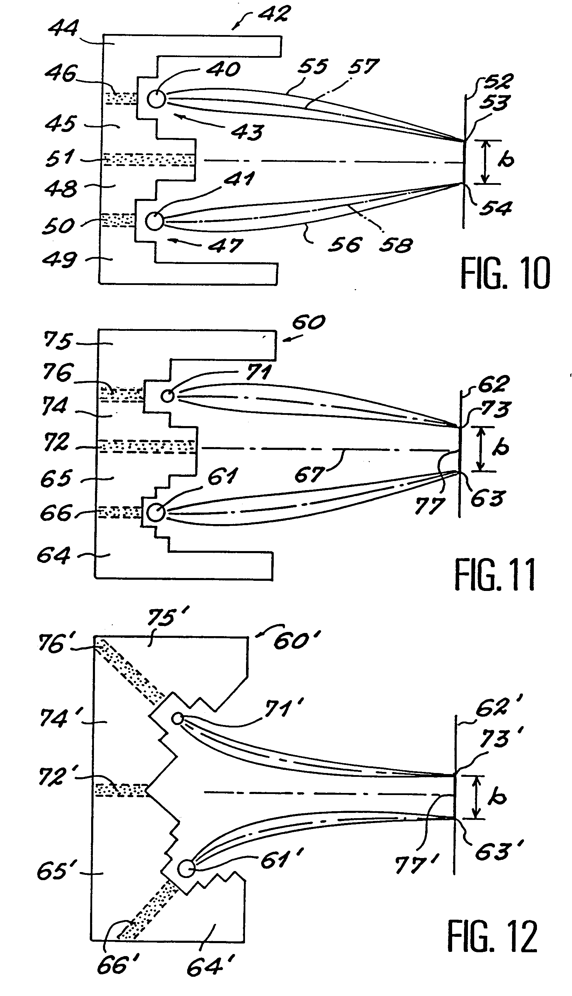

Selon l'invention, il est préférable d'utiliser une seule cathode comportant deux filaments. Trois exemples de réalisation d'une telle cathode seront décrits en relation avec les figures 10, 11 et 12.

Dans l'exemple de réalisation de la figure 10, une cathode 42 comprend deux filaments identiques 40 et 41 qui sont disposés dans une pièce de concentration divisés en deux parties identiques, l'une pour focaliser les électrons émis par le filament 40 et l'autre pour focaliser les électrons émis par le filament 41.

C'est ainsi que le filament 40 est situé au fond d'une rainure 43 en forme de marches d'escalier qui est réalisée en deux parties métalliques 44 et 45 accolées mais isolées l'une de l'autre par une cloison ou couche isolante 46.

De même le filament 41 est situé au fond d'une rainure 47 en forme de marches d'escalier qui est réalisée en deux parties métalliques 48 et 49 accolées mais isolées l'une de l'autre par une couche isolante 50. Les parties métalliques centrales 45 et 48 sont isolées l'une de l'autre par une couche isolante 51. La pièce de concentration comporte ainsi quatre parties métalliques 44, 45, 48 et 49 accolées mais isolées l'une par rapport à l'autre. Cette disposition permet d'appliquer des tensions différentes sur les différentes pièces métalliques et donc de modifier la focalisation des faisceaux d'électrons 55 et 56, c'est-à-dire les dimensions de leur point d'impact 53 et 54 sur l'anode 52 et notamment la dimension a. En outre, elle permet aussi de modifier la position angulaire de l'axe d'émission 57 et 58 desdits faisceaux pour modifier la distance b entre les points d'impact 53 et 54.

Alors que la cathode décrite en relation avec la figure 10 est satisfaisante pour mettre en oeuvre l'invention, elle n'est pas suffisamment sophistiquée pour réaliser certaines autres fonctions mises en oeuvre dans les appareils radiologiques, telles que l'obtention de rayonnements X de caractéristiques différentes. A cet effet, il est connu d'utiliser des cathodes comportant deux filaments qui ont des caractéristiques différentes et qui sont utilisées successivement, c'est-à-dire de manière non simultanée. Pour continuer à réaliser les fonctions requises tout en mettant en oeuvre la présente invention, ces cathodes doivent être modifiées et les figures 11 et 12 montrent des exemples de telles modifications qui découlent de l'exemple de réalisation décrit en relation avec la figure 10.

Sur la figure 11, une cathode 60 comprend un premier filament 61 dont les dimensions sont telles qu'il donne naissance sur une anode 62 à un premier foyer 63 dit "Grand foyer". Les électrons émis par le premier filament 61 sont focalisés et dirigés vers le foyer 63 par un premier dispositif de focalisation qui comporte deux pièces métalliques 64 et 65 accolées mais isolées l'une de l'autre par une couche isolante 66. Ces deux pièces 64 et 65 réalisent autour du filament 61, une pièce de concentration en forme bien connue de trois marches d'escalier.

La cathode 60 comprend un deuxième filament 71 dont les dimensions sont inférieures à celles du premier filament 61 et qui donne naissance sur l'anode 62 à un deuxième foyer 73 dit "Petit foyer". Les électrons émis par ce deuxième filament 71 sont focalisés et dirigés vers le foyer 73 par un deuxième dispositif de focalisation qui comporte deux pièces métalliques 74 et 75 accolées mais isolées l'une de l'autre par une couche isolante 76. Ces deux pièces métalliques 74 et 75 réalisent autour du filament 71 une pièce de concentration en forme de deux marches d'escalier.

Les deux dispositifs de focalisation sont accolées par les parties métalliques 65 et 74 qui sont isolées l'une de l'autre par une couche isolante 72.

Le nombre de marches de chaque dispositif de focalisation ainsi que la hauteur desdites marches sont différents afin d'obtenir la focalisation et la déviation recherchées des faisceaux d'électrons dont l'intensité est généralement différente car les caractéristiques des filaments sont différentes : dimensions, longueurs, résistances, courants de chauffage. Les caractéristiques mécaniques des dispositifs de focalisation ne sont en général pas suffisantes pour obtenir les effets recherchés, aussi il est prévu d'appliquer des tensions différentes aux pièces métalliques 64,65,74 et 75 et c'est la raison de leur isolation électrique l'une par rapport à l'autre. La réalisation d'une telle cathode est à la portée de l'homme de métier sans faire oeuvre d'invention.

L'exemple de réalisation de la figure 12 est similaire à celui de la figure 11 avec cette différence que les axes des dispositifs de focalisation ne sont pas parallèles entre eux comme sur la figure 11 mais sont sécants et l'on obtient ce qui est appelé une cathode en forme de dièdre. Sur cette figure 12, les éléments similaires à ceux de la figure 11 portent la même référence mais affectés d'un indice "'".

Dans les tubes à rayons X à deux foyers utilisés dans les appareils radiologiques de type classique, on n'utilise qu'un foyer à la fois et il suffit de focaliser l'un des deux faisceaux d'électrons en un point 77 qui sera le même pour les deux faisceaux.

Pour utiliser un tel tube à rayons X afin de mettre en oeuvre la présente invention, il est nécessaire d'obtenir simultanément deux foyers 63 et 73 qui soient aussi identiques que possible et qui soient séparés par une distance déterminée b.

A cet effet, les caractéristiques mécaniques et électriques des dispositifs de focalisation de chaque filament ainsi que les valeurs des courants de chauffage desdits filaments sont déterminés pour permettre la modification de la focalisation des faisceaux afin qu'ils donnent naissance à des foyers identiques et la modification de leur déviation pour obtenir la distance b entre les foyers.

Il est important de pouvoir modifier la distance afin de tenir compte du fait que le grandissement G de l'appareil radiologique change selon le type de pose.

Pour déterminer les potentiels à appliquer aux pièces métalliques des différents dispositifs de focalisation et de déflexion des cathodes des figures 10,11 et 12, il est nécessaire d'effectuer des étalonnages du tube à rayons X comportant ladite cathode en faisant varier les tensions U appliquées aux différentes parties métalliques. Ces étalonnages ont également été décrits dans la demande de brevet français 90 01249 déposée le 2 février 1990 mais seront à nouveau décrits ici dans leur application à la cathode 60, de la figure 11.

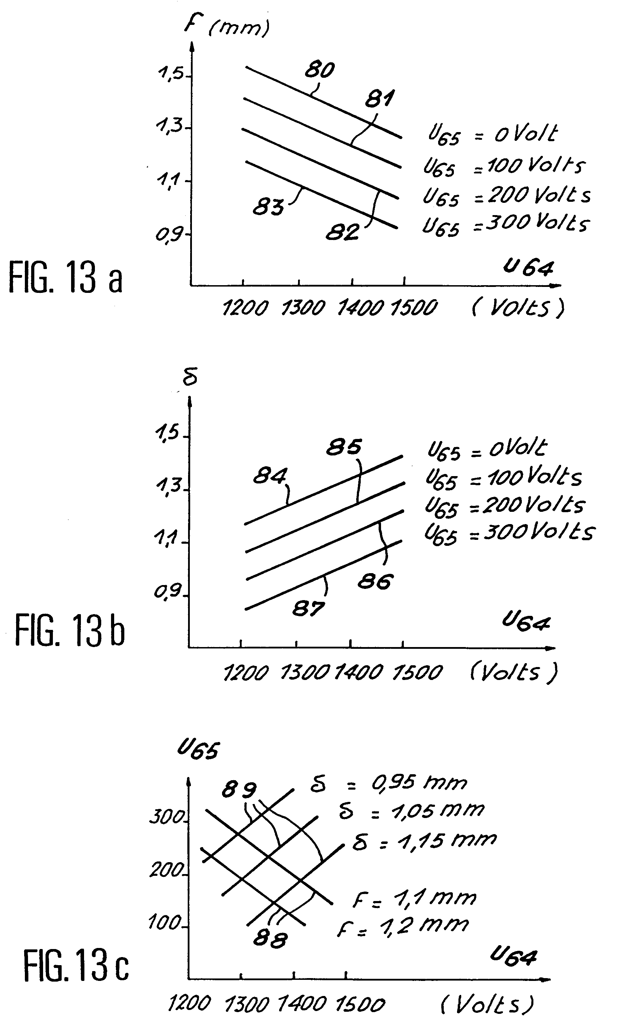

Un premier étalonnage consiste à mesurer la largeur f du point d'impact du faisceau d'électrons sur l'anode 62, c'est-à-dire la largeur du foyer du faisceau de rayons X, en fonction du potentiel $U_{64}$ appliqué à la partie 64 pour différentes valeurs du $U_{65}$ appliqué à la partie 65. On obtient les courbes 80 à 83 de la figure 13a qui correspondent respectivement à $U_{65}$ = 0 volt, -100 volts, -200 volts et -300 volts.

Un deuxième étalonnage consiste à mesurer la déflexion $\delta$ du faisceau d'électrons, c'est-à-dire le déplacement de son point d'impact 63 sur l'anode 62 par rapport à un plan ou axe médian 67, en fonction du potentiel $U_{64}$ et pour les mêmes valeurs de $U_{65}$. On obtient les courbes 84 à 87 de la figure 13b qui correspondent respectivement à $U_{65}$ = 0 volts, -100 volts, -200 volts et -300 volts.

La combinaison des courbes des figures 13a et 13b permet d'obtenir les deux réseaux de courbes de la figure 13c, c'est-à-dire $U_{65}$ en fonction de $U_{64}$ pour différentes valeurs de la largeur f du point d'impact (réseau 88) et pour différentes valeurs de la déflexion $\delta$ du faisceau (réseau 89). Sur cette figure 13c, les coordonnées des points d'intersection des courbes des deux réseaux 88 et 89 donnent les valeurs de $U_{64}$ et $U_{65}$ pour obtenir la largeur du foyer et de sa déflexion indiquées par les courbes sécantes.

Bien entendu, ces étalonnages décrits ci-dessus sont également à effectuer pour le faisceau contrôlé par les pièces 74 et 75.

En résumé, pour une grille anti-diffusante donnée, c'est-à-dire une fréquence spatiale $F_g$ donnée, la formule (1) permet de déterminer les distances b à réaliser entre les deux foyers pour différentes valeurs du grandissement G qui varie habituellement entre 1,05 et 1,2 car au-delà de cette dernière valeur, le rayonnement diffusé est éliminé par suite du trajet dans l'air de sorte que la grille anti-diffusante n'est plus utilisée.

Ensuite, les courbes de la figure 13c permettent de déterminer les potentiels à appliquer aux différentes parties des dispositifs de focalisation et de déflexion pour obtenir, d'une part, chaque espacement b = $2\delta$ entre les foyers 63 et 73, soit une déflexion de b/2 par dispositif, et d'autre part, la même largeur a = f par foyer.

Par ailleurs, les caractéristiques de chaque filament permettent de déterminer le courant de chauffage pour obtenir la même énergie par foyer si on utilise des filaments différents.

Pour un appareil radiologique donné, ces différentes informations sont par exemple enregistrées dans la mémoire d'un microprocesseur qui, pour une certaine valeur du grandissement, donne les valeurs des potentiels à appliquer aux différentes parties métalliques de la cathode ainsi que les valeurs des courants de chauffage des filaments afin d'obtenir les deux foyers de largeur a espacés de la distance b.

Le système selon l'invention pour éliminer l'image de la grille anti-diffusante comporte donc comme le montre la figure 14, un tube 90 à rayons X à deux foyers A et B dont la cathode comporte deux filaments identiques ou non qui sont associés chacun à un dispositif de focalisation et de déflexion. Les différentes tensions d'alimentation et de polarisation de ce tube 90 sont fournies par un dispositif 91 qui est sous le contrôle d'un microprocesseur 92. Ce microprocesseur comporte au moins une mémoire 93 qui contient les valeurs des tensions à appliquer au tube 90 ainsi que les courants de chauffage des filaments pour obtenir simultanément deux foyers identiques de largeur a et d'espacement b qui permettent d'éliminer l'image de la grille anti-diffusante fixe pour un grandissement donné G.

Pour obtenir les déviations requises des faisceaux d'électrons, il a été décrit des exemples de réalisation dans lesquels les déviations étaient obtenues en appliquant des tensions de polarisation aux différentes parties des pièces de focalisation. Ces déviations peuvent être aussi obtenues par d'autres dispositifs tels que, par exemple, des électrodes supplémentaires qui seraient placées de part et d'autre de chaque faisceau et seraient isolées électriquement des pièces de focalisation. Ces pièces seraient portées par lesdites pièces de focalisation et constitueraient des prolongements des marches d'escalier. L'utilisation de telles électrodes supplémentaires permettrait de scinder les deux fonctions : l'une de focalisation réservée au pièces de focalisation à proximité des filaments et l'autre de déviation réservée à ces électrodes supplémentaires placées le long des faisceaux.

## Revendications

1. Dans un appareil radiologique comportant au moins une source (A, 53,73,73') de rayons X irradiant un objet (16,30) à examiner qui est situé à une distance c de ladite source (10,90) et une grille anti-diffusante (19) de fréquence spatiale $F_g$ disposée entre ledit objet (16,30) et un récepteur (14) situé à une distance d dudit objet (16,30), un procédé permettant d'éliminer l'image de ladite grille anti-diffusante (19) qui consiste :
   - à maintenir fixe la grille anti-diffusante (19),
   - à irradier simultanément ledit objet (16,30) par une deuxième source (B, 54,63,63') de rayons X ayant une répartition énergétique spatiale sensiblement identique à celle de la première source, et
   - à choisir la distance b entre les deux sources de rayons X pour que

$$b = \frac{G}{G-1} \cdot \frac{1}{2F_g} \quad (1)$$

avec le grandissement $G = \dfrac{c + d}{c}$

2. Procédé selon la revendication 1, caractérisé en ce qu'il consiste en outre :
   - à modifier la distance b entre les deux sources de rayons X lorsque le grandissement G varie de manière à toujours avoir l'égalité donnée par :

$$b = \frac{G}{G-1} \cdot \frac{1}{2F_g} \quad (1)$$

3. Tube à rayons X pour mettre en oeuvre le procédé des revendications 1 ou 2, caractérisé en ce qu'il comprend une cathode (42,60,60') comportant deux filaments (40,47 ou 71,61 ou 71',61') émettant chacun un faisceau d'électrons, chaque filament étant associé à un dispositif de focalisation et de déviation dont les caractéristiques mécaniques et électriques ainsi que les tensions appliquées sont telles que les deux faisceaux d'électrons donnent naissance, sur une anode (52,62,62') disposée en face de ladite cathode, à deux sources (53,54 ou 73,63 ou 73',63') de rayons X, les deux sources de rayons X ainsi obtenues ayant des répartitions énergétiques spatiales aussi identiques que possible et étant séparées par la distance b.

4. Tube selon la revendication 3, caractérisé en ce que les deux filaments (40,41) ainsi que les deux dispositifs de focalisation et de déviation (44,45,46 et 48,49,50) sont identiques, chaque dispositif de focalisation et de déviation comportant deux pièces métalliques (44,45 ou 48,49) qui sont disposées de part et d'autre du filament associé et isolées électriquement l'une de l'autre par une cloison (46 ou 50) de manière à pouvoir être polarisées à des potentiels différents pour obtenir sur l'anode (52) les deux sources de rayons X séparées par la distance b.

5. Tube selon la revendication 3, caractérisé en ce qu'il comprend un premier filament (71 ou 71') prévu pour donner une première source de rayons X (73 ou 73') ayant des dimensions déterminées, un deuxième filament (61 ou 61') prévu pour donner naissance à une deuxième source de rayons X (63 ou 63') ayant des dimensions plus grandes que la première source, un premier dispositif de focalisation et de déviation (74,75,76 ou 74', 75', 76') associé au premier filament (71 ou 71') pour focaliser et dévier le faisceau d'électrons émis par le premier filament en un premier point (73 ou 73') de l'anode (62 ou 62') et un deuxième dispositif de focalisation et de déviation (64,65,66 ou 64',65',66') associé au deuxième filament (61 ou 61') pour focaliser et dévier le faisceau d'électrons émis par le deuxième filament en un deuxième point (63 ou 63') de l'anode (62 ou 62'), chaque premier et deuxième dispositif de focalisation et de déviation comportent deux pièces métalliques (74,75 ou 74',75', 64,65 ou 64',65') qui sont disposées de part et d'autre du filament associé et isolées électriquement l'une de l'autre par une cloison (76 ou 76', 66 ou 66') de manière à pouvoir être polarisées à des potentiels différents pour obtenir sur l'anode (62 ou 62') des sources de rayons X ayant des dimensions sensiblement identiques et une distance b entre lesdites première et deuxième sources de rayons X, lesdits premier et deuxième filaments étant alimentés par des courants dont les intensités conduisent à des répartitions énergétiques sensiblement identiques pour lesdites sources de rayons X.

6. Système pour obtenir deux sources de rayonnement X de dimensions sensiblement identiques et séparées par une distance b variable caractérisé en ce qu'il comprend un tube 90 à rayons X comportant deux sources de rayonnement simultanées selon l'une des revendications 3,4 ou 5, un dispositif d'alimentation (91) pour générer les tensions d'alimentation et de polarisation dudit tube à rayons X et un microprocesseur (92) pour commander ledit dispositif d'alimentation (91), ledit microprocesseur comportant au moins une mémoire dans laquelle sont enregistrées, pour chaque distance b ou grandissement G, les valeurs desdites tensions d'alimentation et de polarisation, ladite mémoire étant adressée pour la valeur de b ou du grandissement G.

FIG. 1

FIG. 2

FIG. 3

FIG. 14

FIG. 4

FIG. 5

FIG. 6

FIG. 7

FIG. 8

FIG. 9

FIG. 10

FIG. 11

FIG. 12

F (mm)

1,5
1,3
1,1
0,9

80
81

$U_{65}$ = 0 Volt
$U_{65}$ = 100 Volts
$U_{65}$ = 200 Volts
$U_{65}$ = 300 Volts

83
82

$U_{64}$

1200 1300 1400 1500    ( Volts )

**FIG. 13 a**

δ

1,5
1,3
1,1
0,9

85
84

$U_{65}$ = 0 Volt
$U_{65}$ = 100 Volts
$U_{65}$ = 200 Volts
$U_{65}$ = 300 Volts

86
87

$U_{64}$

1200 1300 1400 1500    (Volts )

**FIG. 13 b**

$U_{65}$

300
200
100

89

δ = 0,95 mm
δ = 1,05 mm
δ = 1,15 mm

F = 1,1 mm
F = 1,2 mm

88

$U_{64}$

1200 1300 1400 1500    ( Volts )

**FIG. 13 c**

Office européen
des brevets

# RAPPORT DE RECHERCHE EUROPEENNE

Numero de la demande

EP    91 40 3530

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | FR-A-2 564 996 (M.DUTILLEUX)<br>* page 1, ligne 1 - page 2, ligne 28; figures 1,2 *<br>--- | 1,3 | G03B42/02<br>H01J35/06<br>G21K1/02 |
| A | US-A-4 380 086 (R.J.VAGI)<br>* colonne 1, ligne 1 - colonne 3, ligne 8; figures 1,3 *<br>--- | 1 | |
| A | DE-A-2 207 052 (PHILIPS PATENTVERWALTUNG GMBH)<br>* page 1 - page 6; revendication 1; figures *<br>--- | 1 | |
| A | EP-A-0 182 637 (PICKER INTERNATIONAL, INC.)<br>* revendication 1; figures *<br>--- | 3 | |
| A | FR-A-2 536 583 (ELSCINT, INC.)<br>* abrégé; revendications 1,3; figures *<br><br>----- | 3,4 | |
| | | | **DOMAINES TECHNIQUES RECHERCHES (Int. Cl.5 )**<br><br>G03B<br>H01J<br>A61B<br>G21K<br>G01N |

Le présent rapport a été établi pour toutes les revendications

| Lieu de la recherche | Date d'achèvement de la recherche | Examinateur |
|---|---|---|
| LA HAYE | 03 AVRIL 1992 | HERYET C.D. |